(19)

**Europäisches Patentamt**
**European Patent Office**
**Office européen des brevets**

(11)  **EP 4 368 697 A1**

(12)  **EUROPEAN PATENT APPLICATION**
published in accordance with Art. 153(4) EPC

(43) Date of publication:
**15.05.2024  Bulletin 2024/20**

(21) Application number: **22854962.2**

(22) Date of filing: **28.04.2022**

(51) International Patent Classification (IPC):
*C12N 1/06* (2006.01)    *C12N 1/16* (2006.01)
*C12P 19/08* (2006.01)    *A23K 10/16* (2016.01)
*A23K 10/14* (2016.01)    *C12R 1/865* (2006.01)

(52) Cooperative Patent Classification (CPC):
**A23K 10/14; A23K 10/16; C12N 1/06; C12N 1/16;**
**C12P 19/08; C12R 2001/865**

(86) International application number:
**PCT/CN2022/089879**

(87) International publication number:
**WO 2023/015965 (16.02.2023 Gazette 2023/07)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority:  **11.08.2021   CN 202110917200**

(71) Applicant: **Angel Yeast Co., Ltd.**
**Hubei 443003 (CN)**

(72) Inventors:
• **QIN, Xianwu**
**Yichang, Hubei 443003 (CN)**

• **HU, Junpeng**
**Yichang, Hubei 443003 (CN)**
• **XIE, Zhiwen**
**Yichang, Hubei 443003 (CN)**
• **DAI, Jinjun**
**Yichang, Hubei 443003 (CN)**
• **HUANG, Xin**
**Yichang, Hubei 443003 (CN)**
• **GONG, Fayuan**
**Yichang, Hubei 443003 (CN)**
• **XU, Jie**
**Yichang, Hubei 443003 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

(54)  **HIGH IMMUNE YEAST CELL WALL, AND PREPARATION METHOD THEREFOR AND USE THEREOF**

(57)     Provided are a high immune yeast cell wall, and a preparation method therefor and the use thereof. For the high immune yeast cell wall, the dissolution rate is ≥ 40%, glucan with a relative molecular weight of 80-200 kDa accounts for more than 99% of the total mass of glucan, and the content of glucan is 20%-40% on the basis that the mass of the high immune yeast cell wall is 100%. The high immune yeast cell wall has an improved dissolution rate and immune efficacy, is derived from a yeast, and can be used as a raw material or additive of feed.

EP 4 368 697 A1

**Description**

**Technical Field**

The present invention relates to the field of microbial applications, and in particular related to a high immune yeast cell wall, and a preparation method therefor and use thereof.

**Background Art**

Yeast cell wall, derived from yeast, is a kind of functional green additive or raw material of feed, which is rich in glucan and mannan, and has biological effects of enhancing immunity, promoting growth, relieving stress, adsorbing mycotoxin, adsorbing pathogenic bacteria and resisting tumor. A large number of studies at home and abroad have found that the biological efficacy of yeast cell wall polysaccharides is closely related to its structure, in which the mannan in the yeast cell wall is very similar to the receptor of pathogenic bacteria on the animal intestinal wall, and prevents the adhesion and reproduction of pathogenic bacteria in the intestinal tract after binding with the lectin of pathogenic bacteria. Molecular weight is another important factor affecting the biological activities of $\beta$-1,3-glucan, such as anti-tumor and immune regulation. Polysaccharides with a molecular weight of 100-200 kDa have strong biological activity. Glucan with a molecular weight of more than 90 kDa can form a unique and highly ordered triple helix structure, which is conducive to binding to the receptor and producing biological effects, which is essential for playing an immune role. Meanwhile, the triple helix structure of yeast glucan facilitates the adsorption of mycotoxins by intermolecular interactions including hydrogen bonding and Van der Waals' force.

At present, the common yeast cell wall products on the market are by-products of the production of yeast extracts. The glucan molecular weight is more than 200 kDa, and the water solubility is poor, which limits the use of yeast cell wall in animal breeding, animal health care, plant protection, human nutrition, and many other fields. Physical modification method, chemical modification method, and biological enzyme method were used to improve the dissolution rate of yeast cell wall and reduce the molecular weight of glucan, but the physical modification method was uncertain and the product quality could not be controlled. The chemical modification method uses a large number of chemical reagents, some of which are highly toxic and easily cause environmental pollution, and the chemical modification reaction is violent and easily destroys the functional molecular structure of polysaccharide. The bioenzymatic reaction is mild, but the increase in dissolution rate is limited.

Therefore, it is necessary to carry out deep processing such as enzymolysis and acidolysis on yeast cell wall products to improve the dissolution rate of the yeast cell wall, focus on specific molecular weight fragments 80-200 kDa, improve the immune efficacy of yeast cell wall and broaden the application field of the yeast cell wall.

**Summary of the Invention**

The technical problem to be solved by the present invention is: the lack of a high immune yeast cell wall, the high immune yeast cell wall has a high dissolution rate, and over 99% of the total mass of glucan is glucan with a relative molecular weight of 80-200 kDa.

In view of the deficiencies of the prior art, the first object of the present invention is to provide a high immune yeast cell wall; the second object of the present invention is to provide a preparation method for the above-mentioned high immune yeast cell wall; the third object of the present invention is to provide the use of the above-mentioned high immune yeast cell wall or the high immune yeast cell wall prepared by the above-mentioned preparation method in feed; and the fourth object of the present invention to provide a feed comprising the high immune yeast cell wall described above.

The technical solution of the present invention:
The present invention provides a high immune yeast cell wall, wherein the relative molecular weight of 80-200 kDa accounts for more than 99% of the total mass of glucan, and the content of glucan is 20-40% on the basis that the mass of the high immune yeast cell wall is 100%.

Preferably, the dissolution rate of the above-mentioned high immune yeast cell wall is $\geq$ 40%.

Preferably, the above-mentioned high immune yeast cell wall has a manno oligosaccharide content of $\geq$ 20% based on taking the mass of the high immune yeast cell wall as 100%.

The present invention also provides a preparation method for the above-mentioned high immune yeast cell wall, comprising the following steps of:

(1) subjecting a raw material containing yeast to autolysis to break the wall, isolating to obtain the yeast cell wall milk;
(2) subjecting the yeast cell wall milk obtained in step (1) to protease enzymolysis and secondary enzymolysis successively, wherein the enzyme used in the secondary enzymolysis is one or a combination of two or more selected from the group consisting of glucanase, mannanase, cellulase, and amylase;

(3) subjecting the enzymatic hydrolysate obtained in step (2) to acidolysis to obtain a high immune yeast cell wall.

Preferably, the autolysis to break the wall in step (1) is carried out at a salt concentration of 2.0-5.5%, a pH of 4.0-6.5, and a temperature of 45-75°C, preferably for an autolysis time of 15-30 h.

Preferably, the protease in step (2) is added in an amount of 1-10‰ based on the dry matter mass of the yeast cell wall milk, preferably, the protease is one or a combination of two or more selected from the group consisting of papain, neutral protease, alkaline protease, and bromelain, preferably the protease comprises neutral protease and bromelain, further preferably, the neutral protease is added in an amount of 2-5%o and the bromelain is added in an amount of 2-5%o.

Preferably, the proteases are neutral protease and bromelain, or neutral protease, bromelain and papain, or papain, neutral protease, alkaline protease and bromelain.

Preferably, the protease is added in an amount of 6-10‰.

Preferably, the enzymolysis temperature of the protease is 30-60°C, the enzymolysis pH is 4.5-7.0 and the enzymolysis time is 6-10 h.

Preferably, based on the dry matter mass of the yeast cell wall milk, the enzyme used in the secondary enzymolysis is added in an amount of 1-10‰, preferably, the secondary enzymolysis temperature is 40-60°C, the enzymolysis pH is 4.0-7.0, and the enzymolysis time is 4-12 h.

Preferably, the enzyme used in the secondary enzymolysis comprises a glucanase and a cellulase, preferably, the glucanase is added in an amount of 2-3%o and the cellulase is added in an amount of 2-3‰.

Preferably, the enzyme used in the secondary enzymolysis is glucanase and cellulase, or glucanase, mannanase and cellulase, or glucanase, mannanase, cellulase and amylase.

Preferably, the enzyme used in the secondary enzymolysis is added in an amount of 4-8%o.

Preferably, the acidolysis pH in step (3) is 1.5-3.5, the acidolysis temperature is 50-100°C, and the acidolysis time is 6-20 h, preferably, the acid used in the acidolysis is an inorganic acid and/or organic acid; further preferably, the inorganic acid is one or a combination of two or more selected from the group consisting of hydrochloric acid, sulfuric acid and phosphoric acid, still more preferably, the organic acid is one or a combination of two or more selected from the group consisting of citric acid, malic acid, lactic acid, formic acid, acetic acid and propionic acid.

Preferably, the above-mentioned preparation method further comprises the step of drying the high immune yeast cell wall: after the completion of acidolysis, the pH value is adjusted to 4.0-7.0, and dried to obtain a powdery high immune yeast cell wall.

Preferably, in step (1), the yeast-containing raw material is obtained by fermentation of a Saccharomyces cerevisiae strain, preferably the Saccharomyces cerevisiae strain is Saccharomyces cerevisiae strain FX-2 (*Saccharomyces cerevisiaed*).

Preferably, the above-mentioned fermentation pH is 4.0-7.0, the fermentation temperature is 28-35°C, and the fermentation time is 15-35 h.

Preferably, the above-mentioned fermentation is performed by feeding a carbon source, a nitrogen source, and a phosphorus source in a fed-batch manner;

the carbon source is 6,000-8,000 parts, the nitrogen source is 400-700 parts, and the phosphorus source is 300-600 parts;

the carbon source is one or a combination of two or more selected from the group consisting of molasses, corn starch, glucose, maltose, trehalose, trehalose, and galactose, preferably molasses; the nitrogen source is selected from one of urea, ammonia, or ammonium sulphate, preferably urea;

the phosphorus source is selected from one of phosphoric acid or potassium dihydrogen phosphate, preferably potassium dihydrogen phosphate.

The present invention also provides the use of the above-mentioned high immune yeast cell wall or the high immune yeast cell wall prepared by the above-mentioned preparation method in feed.

The present invention also provides a feed comprising the above-mentioned high immune yeast cell walls or the high immune yeast cell walls prepared by the above-mentioned preparation method and basal diets, the high immune yeast cell wall is added to the feed in an amount of 0.5-10 wt%o.

Advantageous effects of the invention:

(1) the high immune yeast cell wall of the present invention has a high dissolution rate, and over 99% of the total mass of glucan is glucan with a relative molecular weight of 80-200 kDa, so the immune efficacy is significantly improved; it is derived from yeast and is a green and environmentally friendly feed raw material or additive;
(2) in the present invention, the secondary enzymolysis and acidolysis processes are used, the preparation process is environmentally friendly, and the solubility of yeast cell wall and the degradation of glucan are improved, at the same time, the functional molecular structure of polysaccharide is more retained, the immune efficacy of yeast cell wall and its added value are improved, and the application field of yeast cell wall is widened.

**Detailed Description of the Invention**

It should be noted that the embodiments and features of the embodiments in the present application can be combined with each other without conflict. Hereinafter, the present invention will be described in detail with reference to examples.

The present invention provides a high immune yeast cell wall, wherein the relative molecular weight of 80-200 kDa accounts for more than 99% of the total mass of glucan, and the content of glucan is 20-40% on the basis that the mass of the high immune yeast cell wall is 100%.

In a preferred embodiment of the present invention, the dissolution rate of the above-mentioned high immune yeast cell wall is $\geq 40\%$.

Compared to the existing yeast cell walls, the dissolution rate is increased, the molecular biological activity of glucan is increased, and the immune efficacy of the yeast cell walls is increased.

In a preferred embodiment of the present invention, the high immune yeast cell wall has a manno oligosaccharide content of $\geq 20\%$ based on taking the mass of the above-mentioned high immune yeast cell wall as 100%.

The present invention also provides a preparation method for the above-mentioned high immune yeast cell wall, comprising the following steps of:

(1) subjecting a raw material containing yeast to autolysis to break the wall, isolating to obtain the yeast cell wall milk;
(2) subjecting the yeast cell wall milk obtained in step (1) to protease enzymolysis and secondary enzymolysis successively, wherein the enzyme used in the secondary enzymolysis is one or a combination of two or more selected from the group consisting of glucanase, mannanase, cellulase, and amylase;
(3) subjecting the enzymatic hydrolysate obtained in step (2) to acidolysis to obtain a high immune yeast cell wall.

In a preferred embodiment of the present invention, the autolysis to break the wall in step (1) is carried out at a salt concentration of 2.0-5.5%, a pH of 4.0-6.5, and a temperature of 45-75°C, preferably for an autolysis time of 15-30 h.

In a preferred embodiment of the present invention, the protease in step (2) is added in an amount of 1-10‰ based on the dry matter mass of the yeast cell wall milk, preferably, the protease is one or a combination of two or more selected from the group consisting of papain, neutral protease, alkaline protease, and bromelain; preferably the protease comprises neutral protease and bromelain; further preferably, the neutral protease is added in an amount of 2-5‰ and the bromelain is added in an amount of 2-5‰.

In a preferred embodiment of the present invention, the proteases are neutral protease and bromelain, or neutral protease, bromelain and papain, or papain, neutral protease, alkaline protease, and bromelain.

In a preferred embodiment of the present invention, the protease is added in an amount of 6-10‰. In a preferred embodiment of the present invention, the enzymolysis temperature of the protease is 30-60°C, the enzymolysis pH is 4.5-7.0 and the enzymolysis time is 6-10 h. When the amount of enzyme is insufficient, the efficiency of enzymolysis is not enough, and excessive enzyme addition may lead to excessive enzymolysis, high cost, and cannot meet the product quality requirements.

In a preferred embodiment of the present invention, based on the dry matter mass of the yeast cell wall milk, the enzyme used in the secondary enzymolysis is added in an amount of 1-10‰; preferably, the secondary enzymolysis temperature is 40-60°C, the enzymolysis pH is 4.0-7.0, and the enzymolysis time is 4-12 h. When the amount of enzyme is insufficient, the efficiency of enzymolysis is not enough. The excessive addition of enzymes will lead to excessive enzymolysis, high cost, and cannot meet the product quality requirements.

In a preferred embodiment of the present invention, the enzyme used in the secondary enzymolysis comprises a glucanase and a cellulase, preferably, the glucanase is added in an amount of 2-3‰ and the cellulase is added in an amount of 2-3‰.

In a preferred embodiment of the present invention, the enzyme used in the secondary enzymolysis is glucanase and cellulase, or glucanase, mannanase, and cellulase, or glucanase, mannanase, cellulase, and amylase.

In a preferred embodiment of the present invention, the enzyme used in the secondary enzymolysis is added in an amount of 4-8‰.

In a preferred embodiment of the present invention, the acidolysis pH in step (3) is 1.5-3.5, the acidolysis temperature is 50-100°C, and the acidolysis time is 6-20 h, preferably, the acid used in the acidolysis is an inorganic acid and/or organic acid; further preferably, the inorganic acid is one or a combination of two or more selected from the group consisting of hydrochloric acid, sulfuric acid, and phosphoric acid; still more preferably, the organic acid is one or a combination of two or more selected from the group consisting of citric acid, malic acid, lactic acid, formic acid, acetic acid, and propionic acid.

In a preferred embodiment of the present invention, the above-mentioned preparation method further comprises the step of drying the high immune yeast cell wall: after the completion of acidolysis, the pH value is adjusted to 4.0-7.0, and dried to obtain a powdery high immune yeast cell wall.

In a preferred embodiment of the present invention, in step (1), the yeast-containing raw material is obtained by fermentation of a Saccharomyces cerevisiae strain, preferably the Saccharomyces cerevisiae strain is Saccharomyces cerevisiae strain FX-2 (*Saccharomyces cerevisiaed*), the strain was deposited in China Center for Type Culture Collection (CCTCC) on August 1, 2016 with the deposit number of CCTCC NO: M2016418, deposited at: Wuhan University, Wuhan, China, postal code: 430072, Tel: (027)-68754052; this strain has been described in patent publication No. CN108220175A.

In a preferred embodiment of the present invention, the fermentation pH is 4.0-7.0, the fermentation temperature is 28-35°C, and the fermentation time is 15-35 h.

In a preferred embodiment of the present invention, the fermentation is performed by feeding a carbon source, a nitrogen source, and a phosphorus source in a fed-batch manner;

the carbon source is 6,000-8,000 parts, the nitrogen source is 400-700 parts, and the phosphorus source is 300-600 parts;

the carbon source is one or a combination of two or more selected from the group consisting of molasses, corn starch, glucose, maltose, trehalose, trehalose, and galactose, preferably molasses; the nitrogen source is selected from one of urea, ammonia, or ammonium sulphate, preferably urea;

the phosphorus source is selected from one of phosphoric acid or potassium dihydrogen phosphate, preferably potassium dihydrogen phosphate.

The present invention also provides the use of the above-mentioned high immune yeast cell wall or the high immune yeast cell wall prepared by the above-mentioned preparation method in feed.

The present invention also provides a feed comprising the above-mentioned high immune yeast cell walls or the high immune yeast cell walls prepared by the above-mentioned preparation method and basal diets, the high immune yeast cell wall is added to the feed in an amount of 0.5-10 wt%o.

The benefits of the present invention are further illustrated by the following specific examples. Raw materials and equipment sources used in examples and comparative examples of the present invention are shown in Table 1.

Table 1 Raw material and equipment sources used in examples and comparative examples of the present invention

| Raw materials or equipment used | Model\ Purity | Vendor |
|---|---|---|
| Papain | 500,000 U/g | Nanning Pangbo Biological Engineering Co., Ltd. |
| Alkaline protease | 500,000 U/g | Angel Yeast Co., Ltd. |
| Neutral protease | 300,000 U/g | Angel Yeast Co., Ltd. |
| Bromelain | 500,000 U/g | Nanning Pangbo Biological Engineering Co., Ltd. |
| Glucanase | 80,000 U/g | Angel Yeast Co., Ltd. |
| Mannanase | 25,000 U/g | Angel Yeast Co., Ltd. |
| Cellulase | 50,000 U/g | Angel Yeast Co., Ltd. |
| Amylase | 20,000 U/g | Angel Yeast Co., Ltd. |
| Urea | Food grade | Hubei Xinghengye Technology Co., Ltd. |
| Potassium dihydrogen phosphate | Food grade 99% | Hubei Xingyinhe Chemical Co., Ltd. |
| Molasses | Total sugar content 45%-80% | Chifeng Lantian Sugar Industry Co., Ltd. |
| Sulfuric acid | Food grade | Hubei Zhongyi Environmental Technology Co., Ltd. |
| Citric acid | 99% food grade | Weifang Ensign Industry Co., Ltd. |
| Phosphoric acid | Food grade | Hubei Xingyinhe Chemical Co., Ltd. |

(continued)

| Raw materials or equipment used | Model\ Purity | Vendor |
|---|---|---|
| Acetic acid | Food grade | Weifang Longshengda Chemical Co., Ltd. |

**Example 1**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 1‰ papain (based on the dry matter mass of the yeast cell wall milk, the same below) was added, the temperature was controlled at 30°C, pH4.5, and the enzymolysis was performed for 6 h;

(4) 1 ‰ glucanase (based on the dry matter mass of the yeast cell wall milk, the same as below) was added, the temperature was controlled at 40°C, pH4.0, and the enzymolysis was performed for 4 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

(II) Product assay of high immune yeast cell wall

1. Determination of mannan and glucan

1.1 Detection principle

According to the different partition coefficients of glucan and mannose between the mobile phase and the stationary phase of the liquid chromatography column, inject the hydrolyzed sample into liquid chromatography, use pure water as the mobile phase, flow out the saccharide molecules, detect with the difference detector, and use the external standard method to quantify. In the hydrolysis of glucans and mannans, the detection result is lower than that of glucans and mannans actually contained in the product because the sample may have incomplete hydrolysis and the glucose and mannose produced by hydrolysis partially undergo other side reactions due to high temperature. The glucan reference substance was used in the test to correct the error caused by the above-mentioned hydrolysis process.

1.2 instruments

a) water bath kettle;
b) vortex mixer;
c) electric furnace;
d) pressure steam sterilizer;
e) high-performance liquid chromatograph: band differential detector and sugar column (6.5 mm × 300 mm waters sugar pak -1).

1.3 Reagents

a) pure water;
b) hydrochloric acid: about 37%;
c) glucose: AR;
d) mannose: AR;
e) sodium hydroxide: AR;
f) Glucose and mannose mixed standard solution (2g/L): 0.2000 g of glucose and mannose were weighed, respectively, and diluted to 100 ml with purified water.
g) glucan reference substance (curdlan from Alcaligenes faecalis): Sigma Product, Art.No.
h) Sodium hydroxide solution: 300 g/L.

1.4 Sample treatment

400 mg (accurate to 0.1 mg) of the sample was accurately weighed and placed into a small test tube with a screw cap made of 20 ml heat-resistant glass, 6.0 ml hydrochloric acid (37%) was added, carefully tightening the vial cap, and then a vortex mixer was used to mix them to obtain a uniform suspension. The vials were placed in a 30°C water bath for 45 min and oscillated and mixed with a vortex mixer once every 15 min. The suspension was then quantitatively transferred to a 200 ml Dewar flask, the small test tube was washed with about 100 ml to 120 ml of water in several portions, and the washing solution was incorporated into the Dewar flask. The Dewar flask was placed in an autoclave and processed for 60 min at 121°C. After removal and cooling, the solution was treated with sodium hydroxide solution to adjust the pH to 6-7 and then made up to 200 ml. Cellulose acetate membranes with a pore size of 0.45 microns were used for filtration before use. 200 mg of glucan reference substance was accurately weighed (see g in 1.3), and the same treatment was performed according to the sample treatment method.

1.5 Chromatographic conditions

Pure water was used as the mobile phase, the flow rate was 0.5 ml/min, the column temperature was 80°C, and the sample was injected after the baseline of the instrument was stable.

1.6 Plotting of standard curve

1, 2, 3, 4, and 5 ml of mannose/glucose standard solution (see f) in 1.3) was respectively sucked into a 10 ml volumetric flask, and high-purity water was used to make constant volume to scale, so as to obtain mixed standard samples with mannose and glucose of 200, 400, 600, 800 and 1000 mg/l, respectively. 20 ul of sample was injected accurately under the above-mentioned chromatographic conditions, to obtain the regression equation between the chromatographic peak area and the concentration of standard substance, and the standard curve was plotted.

1.7 Determination of samples and reference substances

Under the same chromatographic conditions, the treated sample and glucan reference substance were injected into the chromatograph respectively, and the retention time and peak area of each chromatographic peak were recorded. Qualifying with the retention time of the chromatographic peak of the sugar standard sample and quantifying with the peak area of the chromatographic peak of the sugar standard sample.

1.8 Result calculation

The content of β-glucan or mannan is calculated according to the following formula:

$$X = (A1 \times 0.2 \times 100) \div (m1 \times 1000) \times 0.9 \times F \ldots\ldots\ldots\ldots(1)$$

$$F = P \times (100-W) \div [(A2 \times 0.2 \times 100) \div (m2 \times 1000) \times 0.9] \ldots (2)$$

wherein:

X---Content of glucan or mannan in the sample, %;
A1---According to the peak area of the sample solution, the glucose or mannose content of the sample solution was found on the standard curve, mg/L;

A2---According to the peak area of glucan reference substance solution, the glucose content of the sample solution was found on the standard curve, mg/L;

m1---Mass of sample weighed, g;

m2 --- Weight of the glucan reference substance, g;

0.2 --- Volume of constant volume after sample/glucan reference substance treatment, L;

0.9---Conversing glucose or mannose to coefficient of glucan or mannan;

F-Experience compensation factor of lower results due to the destruction of glucose and mannose in the acidolysis of the sample;

P---Purity of glucan reference substance (according to the test report provided by the reagent manufacturer);

W--Water content of glucan reference substance (according to the test report provided by the reagent manufacturer).

Comments: in the same laboratory, F value is usually detected every 1-2 months. The F value is about 1.14, and the F value is regularly corrected in the laboratory.

1.9 Allowable error

The relative error of two independent determination results obtained under the conditions of repeated detection shall not exceed the value specified in Table 2:

Table 2 Relative error of repeatability test

| Content | $\leq 10\%$ | 10-30% (without 10% and 30%) | $\geq 30\%$ |
|---|---|---|---|
| Mannan | 10% | 5% | 3% |
| Glucan | 10% | 5% | 3% |

2. Method for measuring dissolution rate

2.1 Principle of determination

The sample was dissolved in water and collected the precipitate by centrifugation, and the weight ratio of the dissolved substance to the total weight was calculated.

2.2 Reagents and instruments

a) distilled water;
b) centrifuge (5,000 g);
c) moisture analyzer;
d) analytical balance;

2.3 Assay procedure

10 g of sample (accurate to 0.1 mg) was accurately weighed, recorded as $m_0$, dissolved in 200 mL of distilled water, transferred into a centrifuge cup after sufficient dissolution, centrifuged for 5 min at 5,000 g, the weight of precipitate was accurately determined and recorded as $m_1$, and the dry substance content of precipitate (make detection according to the Method 1 specified in GB 5009.3-2010) was determined and record as D.

2.4 Result calculation

Sample dissolution rate:

$$X = (m_0 - m_1 \times D) \div m_0 \times 100\%$$

X--Sample dissolution rate, %;

$m_0$--Weigh the sample weight, g;

$m_1$--The weight of precipitate after centrifugation, g;
D--The content of dry substance precipitated after centrifugation, %.

The calculation results are retained to one decimal place.

3. The relative molecular weight of glucan was determined as follows:

Determination methods: the high immune yeast cell wall product prepared in step (I) was prepared into a solution, and the relative molecular weight of glucan was determined by using high-phase liquid chromatography.

Analytical Conditions: Shodex OHpak SB-805HQ gel column (8 mm × 300 mm); detector: differential detector (Optilab rEX), octagonal static laser scattering instrument (DAWN HELLOS), UV detector; detection wavelength: 658 nm (glucan); mobile phase: 0.5 mol/L NaCl solution; column temperature: 25°C; flow rate: 0.5 mL/min; sample injection volume: 20 μL.

High-performance liquid chromatograph: model GPC/RI/MALLS, Waters 515 pump, made by Waters, USA.

Relevant test results were shown in Table 3.

## Example 2

(I) Preparation of high immune yeast cell wall

(1) The culture solution contained a carbon source, a nitrogen source, and a phosphorus source, wherein the carbon source was molasses 6,000 g, the nitrogen source was urea 700 g and the phosphorus source was potassium dihydrogen phosphate 300 g. The culture solution was sterilized at 121°C for 10 min, inoculated with Saccharomyces cerevisiae FX-2 (Saccharomyces cerevisiaed) for fermentation culture, and the fermentation temperature was 35°C, the fermentation time was 35 h, and the fermentation pH was 7.0 to obtain yeast milk;

(2) autolysis treatment was performed on the above-mentioned yeast milk, sodium chloride was added to make the mass concentration of sodium chloride 5.5%, autolyzed at pH6.5 and a temperature of 75°C for 30 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 20%, 10 %o alkaline protease (based on the dry matter mass of the yeast cell wall milk, the same below) was added, the temperature was controlled to 60°C, pH7.0, and the enzymolysis was performed for 10 h;

(4) 10 %o mannanase (based on the dry matter mass of the yeast cell wall milk, the same as below) was added, the temperature was controlled at 60°C, pH7.0, and the enzymolysis was performed for 12 h;

(5) citric acid was added to the enzymolysis solution obtained in step (4), the pH was adjusted to 3.5, the temperature was increased to 100°C, and maintained for 20 h;

(6) after the incubation, the pH was adjusted to 7.0, and then spray-dried to obtain high immune yeast cell wall powder.

It was determined according to the product determination method in Example 1. The relevant test results were shown in Table 3.

## Example 3

(I) Preparation of high immune yeast cell wall

(1) The culture solution contained a carbon source, a nitrogen source, and a phosphorus source, wherein the carbon source was molasses 8000 g, the nitrogen source was urea 550 g and the phosphorus source was potassium dihydrogen phosphate 450 g. The culture solution was sterilized at 121°C for 10 min, inoculated with Saccharomyces cerevisiae FX-2 (Saccharomyces cerevisiaed) for fermentation culture, and the fermentation temperature was 30°C, the fermentation time was 20 h, and the fermentation pH was 5.0 to obtain yeast milk;

(2) autolysis treatment was performed on the above-mentioned yeast milk, sodium chloride was added to make the mass concentration of sodium chloride 3%, autolyzed at pH5.0 and a temperature of 55°C for 20 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 10%, 5 %o of neutral protease (based on the dry matter mass of the yeast cell wall milk, the same below) and 5 %o of bromelain (based on the dry matter mass of the yeast cell wall milk, the same below) were added, the temperature was controlled to 50°C, pH5.0, and the enzymolysis was performed for 8 h;

(4) 5 %o cellulase (based on the dry matter mass of the yeast cell wall milk, the same as below) and 5 %o amylase (based on the dry matter mass of the yeast cell wall milk, the same as below) were added, the temperature was controlled at 50°C, pH5.0, and the enzymolysis was performed for 8 h;

(5) phosphoric acid and acetic acid were added to the enzymolysis solution obtained in step (4), the pH was adjusted to 2.5, the temperature was increased to 80°C, and maintained for 8 h;

(6) after the incubation, the pH was adjusted to 5.0, and then spray-dried to obtain high immune yeast cell wall powder.

It was determined according to the product determination method in Example 1. The relevant test results were shown in Table 3.

**Example 4**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 2 %o neutral protease was added, the temperature was controlled at 40°C, pH6.0, and the enzymolysis was performed for 7 h;

(4) 2 ‰ cellulase was added, the temperature was controlled at 40°C, pH6.0, and the enzymolysis was performed for 6 h;

(5) 10 wt% sulfuric acid was added to the enzymolysis solution obtained in step (4), the pH was adjusted to 2.0, the temperature was increased to 50°C, and maintained for 13 h;

(6) after the incubation, the pH was adjusted to 5.0, and then spray-dried to obtain high immune yeast cell wall powder.

**Example 5**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 5 %o bromelain was added, the temperature was controlled at 45°C, pH5.5, and the enzymolysis was performed for 9 h;

(4) 5 ‰ amylase was added, the temperature was controlled at 50°C, pH4.0, and the enzymolysis was performed for 5 h;

(5) citric acid was added to the enzymolysis solution obtained in step (4), the pH was adjusted to 3.0, the temperature was increased to 60°C, and maintained for 16 h;

(6) after the incubation, the pH was adjusted to 6.0, and then spray-dried to obtain high immune yeast cell wall powder.

**Example 6**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 2 ‰ papain and 2 ‰ alkaline protease were added, the temperature was controlled at 55°C, pH6.5, and the enzymolysis was performed for 8 h;

(4) 2 ‰ glucanase and 2 ‰ cellulase were added, the temperature was controlled at 60°C, pH5.0, and the enzymolysis was performed for 7 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

**Example 7**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 2 ‰ papain, 2 ‰ neutral protease, and 0.2 ‰ bromelain were added, the temperature was controlled at 60°C, pH4.5, and the enzymolysis was performed for 7 h;

(4) 3 ‰ mannanase and 3 ‰ amylase were added, the temperature was controlled at 40°C, pH7.0, and the enzymolysis was performed for 10 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

**Example 8**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 2 ‰ papain, 2 ‰ neutral protease, 2 ‰ bromelain, and 2 ‰ alkaline protease were added, the temperature was controlled at 50°C, pH5.5, and the enzymolysis was performed for 6 h;

(4) 3 ‰ glucanase, 3 ‰ mannanase, and 3 ‰ cellulase were added, the temperature was controlled at 50°C, pH9.0, and the enzymolysis was performed for 9 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

**Example 9**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 3 ‰ papain, 3 ‰ bromelain, and 3 ‰ alkaline protease were added, the temperature was controlled at 45°C, pH5.0, and the enzymolysis was performed for 8 h;

(4) 2 ‰ glucanase, 2 ‰ mannanase, 2 ‰ cellulase, and 2 ‰ amylase were added, the temperature was controlled at 60°C, pH5.0, and the enzymolysis was performed for 11 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

**Comparative Example 1**

(I) Preparation of high immune yeast cell wall

(1) The culture solution contained a carbon source, a nitrogen source and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 0.5 ‰ papain was added, the temperature was controlled at 30°C, pH4.5, and the enzymolysis was performed for 6 h;

(4) 1 %o glucanase was added, the temperature was at 40°C, pH4.0, and the enzymolysis was performed for 4 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

It was determined according to the product determination method in Example 1. The relevant test results were shown in Table 3.

**Comparative Example 2**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 1 %o papain was added, the temperature was controlled at 30°C, pH4.5, and the enzymolysis was performed for 6 h;

(4) 0.5 %o glucanase was added, the temperature was controlled at 40°C, pH4.0, and the enzymolysis was performed for 4 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

It was determined according to the product determination method in Example 1. The relevant test results were shown in Table 3.

**Comparative Example 3**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 35°C, the fermentation time was 35 h, and the fermentation pH was 7.0 to obtain yeast milk;

(2) autolysis treatment was performed on the above-mentioned yeast milk, sodium chloride was added to make the mass concentration of sodium chloride 5.5%, autolyzed at pH6.5 and a temperature of 75°C for 30 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 20%, 12 %o alkaline protease was added, the temperature was controlled at 60°C, pH7.0, and the enzymolysis was performed for 10 h;

(4) 10 %o mannanase was added, the temperature was controlled at 60°C, pH7.0, and the enzymolysis was performed for 12 h;

(5) citric acid was added to the enzymolysis solution obtained in step (4), the pH was adjusted to 3.5, the temperature was increased to 100°C, and maintained for 20 h;

(6) after the incubation, the pH was adjusted to 7.0, and then spray-dried to obtain high immune yeast cell wall powder.

It was determined according to the product determination method in Example 1. The relevant test results were shown in Table 3.

## Comparative Example 4

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 35°C, the fermentation time was 35 h, and the fermentation pH was 7.0 to obtain yeast milk;

(2) autolysis treatment was performed on the above-mentioned yeast milk, sodium chloride was added to make the mass concentration of sodium chloride 5.5%, autolyzed at pH6.5 and a temperature of 75°C for 30 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 20%, 10 ‰ alkaline protease was added, the temperature was controlled at 60°C, pH7.0, and the enzymolysis was performed for 10 h;

(4) 12 ‰ mannanase was added, the temperature was controlled at 60°C, pH7.0, and the enzymolysis was performed for 12 h;

(5) citric acid was added to the enzymolysis solution obtained in step (4), the pH was adjusted to 3.5, the temperature was increased to 100°C, and maintained for 20 h;

(6) after the incubation, the pH was adjusted to 7.0, and then spray-dried to obtain high immune yeast cell wall powder.

It was determined according to the product determination method in Example 1. The relevant test results were shown in Table 3.

## Comparative Example 5

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 1 ‰ papain was added, the temperature was controlled at 30°C, pH4.5, and the enzymolysis was performed for 6 h;

(4) 1‰ glucanase was added, the temperature was controlled at 40°C, pH4.0, and the enzymolysis was performed for 4 h;

(5) the enzymatic hydrolysate obtained in step (4) was heated to 90°C for 1 h and then spray-dried to obtain high immune yeast cell wall powder.

It was determined according to the product determination method in Example 1. The relevant test results were shown in Table 3.

**Comparative Example 6**

(I) Preparation of high immune yeast cell wall

(1) The culture solution contained a carbon source, a nitrogen source, and a phosphorus source, wherein the carbon source was molasses 7,000 g, the nitrogen source was urea 400 g and the phosphorus source was potassium dihydrogen phosphate 600 g. The culture solution was sterilized at 121°C for 10 min, inoculated with Saccharomyces cerevisiae FX-2 (Saccharomyces cerevisiaed) for fermentation culture, and the fermentation temperature was 28°C, fermentation time was 15 h, and fermentation pH was 4.0 to obtain yeast milk;

(2) autolysis treatment was performed on the above-mentioned yeast milk, sodium chloride was added to make the mass concentration of sodium chloride 2%, autolyzed at pH4.0 and a temperature of 45°C for 15 h, and centrifuged at 5,000 rpm to obtain an upper layer of yeast autolysate and a lower layer of yeast cell wall milk, and the yeast cell wall milk was collected for enzymolysis treatment;

(3) the yeast cell wall milk obtained in step (2) was diluted with water to a dry matter mass concentration of 5%, 1 ‰ glucanase was added, the temperature was controlled at 40°C, pH4.0, and the enzymolysis was performed for 4 h;

(4) 1 ‰ papain was added, the temperature was controlled at 30°C, pH4.5, and the enzymolysis was performed for 6 h;

(5) 10 wt% sulfuric acid was added to the enzymolysis solution obtained in step (4), the pH was adjusted to 1.5, the temperature was increased to 70°C, and maintained for 6 h;

(6) after the incubation, the pH was adjusted to 4.0, and then spray-dried to obtain high immune yeast cell wall powder.

It was determined according to the product determination method in Example 1. The relevant test results were shown in Table 3.

Table 3 Test results of Examples 1 to 9 and Comparative Examples 1 to 6

| | Glucan (%) | Manno oligosaccharide (%) | Dissolution rate (%) | Glucan relative molecular weight, Da | Percentage of total mass of glucan (%) | | |
| --- | --- | --- | --- | --- | --- | --- | --- |
| | | | | | Molecular weight 80-200 KDa | Molecular weight ≥ 200 KDa | Molecular weight ≤ 80 KDa |
| Example 1 | 28.1 | 27.2 | 50 | $9.96 \times 10^4$ | 99.62 | | |
| Example 2 | 26.6 | 29.3 | 49.4 | $9.08 \times 10^4$ | 99.69 | | |
| Example 3 | 27.3 | 29.6 | 50.3 | $1.05 \times 10^5$ | 99.62 | | |
| Example 4 | 20.0 | 33.4 | 45.6 | $2.0 \times 10^5$ | 99.63 | | |
| Example 5 | 30.0 | 22.05 | 43.6 | $1.65 \times 10^5$ | 99.32 | | |
| Example 6 | 40.0 | 20.0 | 40.0 | $1.03 \times 10^5$ | 99.56 | | |
| Example 7 | 28.9 | 29.5 | 50.6 | $1.81 \times 10^5$ | 99.37 | | |
| Example 8 | 25.6 | 28.9 | 53.0 | $1.05 \times 10^5$ | 99.67 | | |
| Example 9 | 29.8 | 27.6 | 52.0 | $9.38 \times 10^4$ | 99.65 | | |
| Comparative Example 1 | 28.6 | 26.6 | 35.1 | $3.45 \times 10^5$ | | 99.89 | |
| Comparative Example 2 | 29.7 | 28.3 | 30.5 | $3.02 \times 10^5$ | | 99.63 | |

EP 4 368 697 A1

(continued)

|  | Glucan (%) | Manno oligosaccharide (%) | Dissolution rate (%) | Glucan relative molecular weight, Da | Percentage of total mass of glucan (%) | | |
|---|---|---|---|---|---|---|---|
|  |  |  |  |  | Molecular weight 80-200 KDa | Molecular weight ≥ 200 KDa | Molecular weight ≤ 80 KDa |
| Comparative Example 3 | 26.5 | 30.3 | 52.3 | $5.06 \times 10^4$ |  |  | 98.45 |
| Comparative Example 4 | 28.74 | 24.3 | 53.6 | $5.36 \times 10^4$ |  |  | 99.76 |
| Comparative Example 5 | 28.8 | 27.3 | 20.5 | $3.5 \times 10^5$ |  | 99.72 |  |
| Comparative Example 6 | 27.9 | 29.8 | 38.8 | $2.76 \times 10^5$ |  | 99.65 |  |

As can be seen from Table 3, the high-immune cell wall powders prepared in Examples 1-9 all have a glucan content of 20%-40%, a manno oligosaccharide content of greater than or equal to 20%, a dissolution rate of more than 40%, and the glucan with a relative molecular weight of 80-200 kDa accounts for more than 99% of the total mass of the glucan; the cell wall samples prepared in Comparative Examples 1-6 have no significant difference in the glucan and mannan content compared with those in Examples, but the cell wall dissolution rates of Comparative Examples 1, 2, 5 and 6 are less than 40% and the glucan with a relative molecular weight of more than 200 kDa accounts for more than 99% of the total mass of the glucan. Comparative Example 3 and Comparative Example 4 have a dissolution rate higher than 40%, but glucan having a relative molecular weight of less than 80 KDa accounts for more than 98% of the total mass of glucan.

**Application Test Example**

72 Duroc x Landrace x Large white hybrid weaned piglets with similar body weight and good body condition aged at 28 days were divided into 9 treatment groups according to the principle of similar average body weight and half male and half female, with four replicates in each group, one pigsty in each replicate and one male and one female pig in each pigsty. The test basal diets were formulated according to the weaned piglets' nutritional needs of NRC 2012. See Table 4, the 9 test groups were fed with different yeast cell wall products on the basis of basal diets. See Table 5 for specific grouping and daily diet design.

The daily management was carried out according to the feeding management method of large-scale pig farms. The piglets were fed with the transition diet (basal diet) after they were purchased. The test diets were fed after the start of the test to ensure that the trough feed was sufficient for the piglets to feed freely. The water was supplied by automatic water drinkers, and the indoor temperature was maintained at normal temperature. Immune status of piglets after purchase and admission: immunized with blue ear disease (PRRS) at 28 days, pseudorabies (PR) at 35 days, and hogcholera (HC) at 42 days.

At the end of the test, 5 ml of blood was collected from the vena cava anterior of all fasted piglets, placed into a centrifuge tube, allowed to stand for 30 min, centrifuged at 3,500 r/min for 10 min, collected the serum, and stored at -20°C for the test. Total protein (TP), albumin (ALB), and globulin (GLO) were detected, and the test results are shown in Table 6.

Table 4 Test basal diets and nutrition level

| Diet composition | Content | Nutrition index | Content |
|---|---|---|---|
| Com/% | 67.00 | Net energy (kcal/kg) | 2900.50 |

16

(continued)

| Diet composition | Content | Nutrition index | Content |
|---|---|---|---|
| Soybean meal (46% protein)/% | 23.30 | Crude protein/% | 19.06 |
| Fermented soybean meal/% | 5.88 | Crude ash/% | 5.00 |
| Soybean oil/% | 1.05 | Dry substance/% | 86.5 |
| Fine stone powder/% | 1.00 | Lysine/% | 1.41 |
| Calcium dihydrogen phosphate/% | 0.065 | Methionine + Cystine/% | 0.79 |
| 4% Premix for piglets/% | 0.50 | Threonine/% | 0.87 |
| Salt/% | 0.37 | Digestible phosphorus/% | 0.18 |
| 98.5% Lysine/% | 0.2 | | |
| Choline chloride/% | 0.05 | | |

Table 5 Test design

| Treatment group | Test diet | Test pig (number) |
|---|---|---|
| Test group 1 | Basal diets + 5 wt%o (based on the weight of basal diet and example sample) Example 1 sample | 4 x 2 |
| Test group 2 | Basal diets + 5 wt%o (same as above) Example 2 Sample | 4 x 2 |
| Test group 3 | Basal diets + 5 wt‰ (same as above) Example 3 Sample | 4 x 2 |
| Test group 4 | Basal diets + 5 wt‰ (same as above) Comparative Example 1 Sample | 4 x 2 |
| Test group 5 | Basal diets + 5 wt%o (same as above) Comparative Example 2 Sample | 4 x 2 |
| Test group 6 | Basal diets + 5 wt%o (same as above) Comparative Example 3 Sample | 4 x 2 |
| Test group 7 | Basal diets + 5 wt%o (same as above) Comparative Example 4 Sample | 4 x 2 |
| Test group 8 | Basal diets + 5 wt‰ (same as above) Comparative Example 5 Sample | 4 x 2 |
| Test group 9 | Basal diets + 5 wt‰ (same as above) Comparative Example 6 Sample | 4 x 2 |

Table 6 Test Results

| Treatment group | Total protein | Globulin | Albumin/globulin |
|---|---|---|---|
| Test group 1 | 46.76 ±1.46 | 17.78 ±4.21 | 1.63 ±0.32 |
| Test group 2 | 47.56 ±2.35 | 18.73 ±3.24 | 1.54 ±0.54 |
| Test group 3 | 47.03 ±3.67 | 18.55 ±4.21 | 1.53 ±0.48 |
| Test group 4 | 44.99 ±1.36 | 14.69 ±0.99 | 2.06 ±0.09 |
| Test group 5 | 46.83 ±2.31 | 15.36 ±3.24 | 2.04 ±0.58 |
| Test group 6 | 45.89 ±2.35 | 14.38 ±1.24 | 2.19 ±0.54 |
| Test group 7 | 47.05 ±2.58 | 15.74 ±2.43 | 1.99 ±0.78 |
| Test group 8 | 46.87 ±3.12 | 15.65 ±2.35 | 1.99 ±1.03 |
| Test group 9 | 47.25 ±2.76 | 16.05 ±1.65 | 1.94 ±0.86 |

It can be seen from Table 6 that after 14 days of tests, s in each treatment group, the serum globulin levels from high to low were Example 2, Example 3, Example 1, Comparative Example 6, Comparative Example 4, Comparative Example 5, Comparative Example 2, Comparative Example 1 and Comparative Example 3 in sequence; the albumin/globulin from low to high were Example 3, Example 2, Example 1, Comparative Example 6, Comparative Example 4, Comparative Example 5, Comparative Example 2, Comparative Example 1, Comparative Example 3; which showed that the high immune yeast cell wall prepared in Example 1, Example 2 and Example 3 could significantly improve the immunity of piglets compared with the high immune yeast cell wall prepared in Comparative Examples.

In summary, the high immune yeast cell wall of the present invention has a high dissolution rate, and more than 99% by mass percentage of glucan with a molecular weight of 80-200 kDa can significantly improve the immune efficacy; it is derived from yeast and is a green and environmentally friendly feed raw material or additive.

The foregoing is illustrative of the preferred embodiments of the present invention and is not to be construed as limiting the invention in any way. Thus, it is intended that the present invention cover the modifications, equivalents, and alternatives falling within the spirit and scope of the invention.

**Claims**

1. A high immune yeast cell wall, **characterized in that** a glucan with a relative molecular weight of 80-200 kDa accounts for more than 99% of total glucan mass, and the glucan content is 20-40% based on taking mass of the high immune yeast cell wall as 100%.

2. The high immune yeast cell wall according to claim 1, **characterized in that** the dissolution rate thereof is ≥ 40%.

3. The high immune yeast cell wall according to claim 1 or 2, **characterized in that** the manno oligosaccharide content is ≥ 20% based on taking mass of the high immune yeast cell wall as 100%.

4. A preparation method for the high immune yeast cell wall according to any one of claims 1 to 3, **characterized by** comprising following steps of:

   (1) subjecting a raw material containing yeast to autolysis to break the wall, isolating to obtain a yeast cell wall milk;
   (2) subjecting the yeast cell wall milk obtained in step (1) to protease enzymolysis and secondary enzymolysis successively, wherein the enzyme used in the secondary enzymolysis is one or a combination of two or more selected from the group consisting of glucanase, mannanase, cellulase, and amylase;
   (3) subjecting an enzymatic hydrolysate obtained in step (2) to acidolysis to obtain a high immune yeast cell wall.

5. The preparation method according to claim 4, **characterized in that** the autolysis to break the wall in step (1) is carried out at a salt concentration of 2.0-5.5%, a pH of 4.0-6.5, and a temperature of 45-75°C, preferably for an autolysis time of 15-30 h.

6. The preparation method according to claim 4 or 5, **characterized in that** the protease in step (2) is added in an amount of 1-10‰ based on the dry matter mass of the yeast cell wall milk; preferably, the protease is one or a combination of two or more selected from the group consisting of papain, neutral protease, alkaline protease and bromelain, preferably the protease comprises neutral protease and bromelain; further preferably, the neutral protease is added in an amount of 2-5‰ and the bromelain is added in an amount of 2-5‰.

7. The preparation method according to claim 6, **characterized in that** the proteases are neutral protease and bromelain, or neutral protease, bromelain and papain, or papain, neutral protease, alkaline protease, and bromelain.

8. The preparation method according to claim 7, **characterized in that** the protease is added in an amount of 6-10‰.

9. The preparation method according to any one of claims 4 to 8, **characterized in that** the enzymolysis temperature of the protease is 30-60°C, the enzymolysis pH is 4.5-7.0 and the enzymolysis time is 6-10 h.

10. The preparation method according to any one of claims 4 to 9, **characterized in that** based on the dry matter mass of the yeast cell wall milk, the enzyme used in the secondary enzymolysis is added in an amount of 1-10‰; preferably, the secondary enzymolysis temperature is 40-60°C, the enzymolysis pH is 4.0-7.0, and the enzymolysis time is 4-12 h.

11. The preparation method according to any one of claims 4 to 10, **characterized in that** the enzyme used in the secondary enzymolysis comprises a glucanase and a cellulase; preferably, the glucanase is added in an amount of 2-3‰ and the cellulase is added in an amount of 2-3‰.

**12.** The preparation method according to claim 11, **characterized in that** the enzyme used in the secondary enzymolysis is glucanase and cellulase, or glucanase, mannanase and cellulase, or glucanase, mannanase, cellulase, and amylase.

**13.** The preparation method according to claim 12, **characterized in that** the enzyme used in the secondary enzymolysis is added in an amount of 4-8%o.

**14.** The preparation method according to any one of claims 4 to 13, **characterized in that** the acidolysis pH in step (3) is 1.5-3.5, the acidolysis temperature is 50-100°C, and the acidolysis time is 6-20 h; preferably, the acid used in the acidolysis is an inorganic acid and/or organic acid; further preferably, the inorganic acid is one or a combination of two or more selected from the group consisting of hydrochloric acid, sulfuric acid, and phosphoric acid; still more preferably, the organic acid is one or a combination of two or more selected from the group consisting of citric acid, malic acid, lactic acid, formic acid, acetic acid, and propionic acid.

**15.** The preparation method according to any one of claims 4 to 14, **characterized by** further comprising subjecting the high immune yeast cell wall to a drying step: after the completion of acidolysis, the pH value is adjusted to 4.0-7.0, and dried to obtain powdery high immune yeast cell wall.

**16.** The preparation method according to any one of claims 4 to 15, **characterized in that** the yeast-containing raw material of step (1) is obtained by fermentation of a Saccharomyces cerevisiae strain; preferably, the Saccharomyces cerevisiae is Saccharomyces cerevisiae FX-2 (Saccharomyces cerevisiaed) deposited with the deposit number CCTCC NO: M20016418.

**17.** The preparation method according to claim 16, **characterized in that** the fermentation pH is 4.0-7.0, the fermentation temperature is 28-35°C, and the fermentation time is 15-35 h.

**18.** The preparation method according to claim 16 or 17, **characterized in that** the fermentation is performed by feeding a carbon source, a nitrogen source, and a phosphorus source in a fed-batch manner;

the carbon source is 6,000-8,000 parts, the nitrogen source is 400-700 parts, and the phosphorus source is 300-600 parts; the carbon source is one or a combination of two or more selected from the group consisting of molasses, corn starch, glucose, maltose, trehalose, trehalose and galactose, preferably molasses;
the nitrogen source is selected from one of urea, ammonia, or ammonium sulphate, preferably urea;
the phosphorus source is selected from one of phosphoric acid or potassium dihydrogen phosphate, preferably potassium dihydrogen phosphate.

**19.** Use of the high immune yeast cell wall of any one of claims 1 to 3 or the high immune yeast cell wall prepared by the preparation method of any one of claims 4 to 18 in feed.

**20.** A feed, **characterized by** comprising the high immune yeast cell wall of any one of claims 1 to 3 or the high immune yeast cell wall prepared by the preparation method of any one of claims 4 to 18 and basal diets, wherein the high immune yeast cell wall is added to the feed in an amount of 0.5-10 wt%o.

## INTERNATIONAL SEARCH REPORT

| International application No. |
|---|
| **PCT/CN2022/089879** |

### A. CLASSIFICATION OF SUBJECT MATTER

C12N 1/06(2006.01)i; C12N 1/16(2006.01)i; C12P 19/08(2006.01)i; A23K 10/16(2016.01)i; A23K 10/14(2016.01)i; C12R 1/865(2006.01)i

According to International Patent Classification (IPC) or to both national classification and IPC

### B. FIELDS SEARCHED

Minimum documentation searched (classification system followed by classification symbols)

C12N; C12P; A23K; C12R

Documentation searched other than minimum documentation to the extent that such documents are included in the fields searched

CNTXT, WPABS, WPABSC, OETXT, USTXTC, VEN, DWPI, CJFD; CNKI, 万方数据, WANFANG DATABASE, PubMed, Elsevier Science, ISI WEB of Science: 酵母, 细胞壁, 葡聚糖, 甘露聚糖, 分子量, 分子质量, Da, KD, 饲料, Saccharomyces cerevisiae, Yeast, Cell wall, Glucan, Dextran, Mannan, Molecular weight, Molecular Mass, Feed

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | CN 109207384 A (ANGEL YEAST CO., LTD.) 15 January 2019 (2019-01-15) see claims 1-20, and description, paragraphs [0055]-[0065] | 1-20 |
| A | JP 2002209598 A (KIRIN BREWERY CO., LTD.) 30 July 2002 (2002-07-30) see claims 1-17, table 1 and table 3 | 1-20 |
| A | CN 108041262 A (ZHANJIANG WUZHOU BIOLOGICAL ENGINEERING CO., LTD.; ZHUHAI TIANXIANGYUAN BIOTECHNOLOGY AND DEVELOPMENT CO., LTD.) 18 May 2018 (2018-05-18) see entire document | 1-20 |
| A | CN 112805013 A (ASAHI GROUP HOLDINGS LTD.) 14 May 2021 (2021-05-14) see abstract, and claims 1-13 | 1-20 |
| A | US 2011250235 A1 (GLYKOS FINLAND OY) 13 October 2011 (2011-10-13) see claims 62-81 | 1-20 |
| A | 朱春森 等 (ZHU Chunsen et al.). "酵母细胞壁多糖的研究进展 (Non-official translation: Research Progress on Yeast Cell Wall Polysaccharides)" 江西饲料 (Jiangxi Feed), No. 4, 28 August 2007 (2007-08-28), see pp. 15-17 | 1-20 |

☐ Further documents are listed in the continuation of Box C.  ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 July 2022** | **27 July 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

International application No.

**PCT/CN2022/089879**

| Patent document cited in search report | | | Publication date (day/month/year) | Patent family member(s) | | | Publication date (day/month/year) |
|---|---|---|---|---|---|---|---|
| CN | 109207384 | A | 15 January 2019 | None | | | |
| JP | 2002209598 | A | 30 July 2002 | None | | | |
| CN | 108041262 | A | 18 May 2018 | None | | | |
| CN | 112805013 | A | 14 May 2021 | WO | 2020075424 | A1 | 16 April 2020 |
| | | | | BR | 112021006128 | A2 | 20 July 2021 |
| | | | | JP | 6530846 | B1 | 12 June 2019 |
| | | | | EP | 3865138 | A1 | 18 August 2021 |
| | | | | TW | 202027757 | A | 01 August 2020 |
| US | 2011250235 | A1 | 13 October 2011 | CN | 102257011 | A | 23 November 2011 |
| | | | | FI | 20080665 | A0 | 18 December 2008 |
| | | | | EP | 2370475 | A1 | 05 October 2011 |
| | | | | ES | 2624703 | T3 | 17 July 2017 |
| | | | | WO | 2010070207 | A1 | 24 June 2010 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108220175 A
- GB 500932010 A